# EUROPEAN PATENT SPECIFICATION

(11) **EP 3 087 920 B1**
(45) Date of publication and mention of the grant of the patent: **26.01.2022**
(21) Application number: 14875363.5
(22) Date of filing: 15.12.2014
(51) Int. Cl.: A61B 6/00, G01N 33/483, G01N 1/36, A01K 1/03, G01N 21/64, A61B 5/00, A61B 5/055, A61B 6/04

(54) **IMAGING DEVICE FOR ANIMALS**
BILDGEBUNGSVORRICHTUNG FÜR TIERE
DISPOSITIF D'IMAGERIE POUR ANIMAUX

(30) Priority: 27.12.2013 KR 20130164881
(43) Date of publication of application: 02.11.2016
(73) Proprietor: Vieworks Co., Ltd., Anyang-si, Gyeonggi-do 431-060 (KR)
(72) Inventor: KANG, You Jung, Paju-si Gyeonggi-do 413-965 (KR); SON, Kyung Su, Yongin-si Gyeonggi-do 446-923 (KR); WON, Ho Keun, Hwaseong-si Gyeonggi-do 445-320 (KR); YANG, Han Su, Seoul 150-942 (KR)
(74) Representative: Lahrtz, Fritz
(86) International application number: PCT/KR2014/012370
(87) International publication number: WO 2015/099347

(56) References cited:
- WO-A1-2005/111587
- JP-A- 2004 121 289
- JP-B2- 4 952 784
- JP-U- H0 521 909
- KR-B1- 101 180 384
- US-A1- 2009 280 064
- US-A1- 2009 281 383

## Description

### [Technical Field]

The present invention relates to an imaging device for animal test subjects, and more particularly, to an imaging device for animal test subjects capable of taking images of changes in a test subject while applying an external stimulus to the test subject.

### [Background Art]

Generally, imaging devices for animals are devices used when conducting an experiment on an animal or an organ and a cell thereof for developing a drug or researching a function of a protein. As experimental animals used in the imaging device for animals, the experiment is mainly conducted on a mouse, rat, or the like. After injecting a fluorescence conjugated antibody or a vascular-contrast-medium with respect to a research protein, a fluorescent substance (5-ALA) responding to a cancer tissue, or the like into an experimental animal and taking images thereof, various types of research on a position of the research protein in a body, a shape of blood vessel, a degree of blood flow, a size of cancer tissue or a function of a blood vessel in the cancer tissue, and the like may be conducted.

Image capturing devices for taking images of experimental animals using X-rays, computerized tomography (CT), magnetic resonance imaging (MRI), and the like have been developed. The image capturing is conducted in a state in which an experimental animal is mounted in a darkroom box or a separate space for imaging.

An example of background art of the present invention is disclosed in Korean Patent Laid-Open Publication No. 2011-0058496 (published on June 01, 2011, and entitled X-ray Radiographic Apparatus for Radiography of Small-Sized Laboratory Animal). Further examples can be taken from e.g. WO 2005/111587 A1 which discloses a biological sample detection device having a portion using a flexible material which is limited to both sides for inserting a hand into the inside of a darkroom space, wherein other portions are made of rigid materials such as metal or resin, and wherein a filter for blocking ultraviolet rays is disposed on a top surface, with a lens for magnifying observation being arranged below the filter

### [Disclosure]

### [Technical Problem]

Conventionally, there has been a problem of not being able to obtain an accurate measurement value for influence on and changes in a test subject while treating or applying external stimulus on the test subject such as a part of an animal body. Thus, a need for improving the problem has been demanded.

The present invention is directed to providing an imaging device for animal test subjects as defined in claim 1, which is capable of taking images of influence on and changes in a test subject while treating the test subject serving as an imaging subject.

### [Technical Solution]

One aspect of the present invention provides an imaging device for animal test subjects having the features of claim 1.In addition, it may be preferable that the test subject supporter be installed to be movable in and out of the darkroom box.

In addition, it may be preferable that the test subject supporter move in a sliding manner.

In addition, it may be preferable that the test subject supporter includes a fastening device adapted to fasten the part of the test subject in order to restrict unexpected motion of the animal body, and the fastening device is adapted to be moved and attached at a different position, and the fastening device is detachable.

In addition, it may be preferable that the image capturing device includes at least one of an optical image capturing device, X-rays, computerized tomography (CT), and magnetic resonance imaging (MRI).

In addition, it may be preferable that the invention further includes an excitation energy generation device adapted to emit energy for the image capturing device to acquire an image and which is installed at least at one place of an inside or outside of the darkroom box. In addition, it may be preferable that the invention further includes an outer door adapted to shield the opening to block external light from being incident into the darkroom box.

In addition, it may be preferable that the invention further includes an input unit adapted to receive operational signals for operating the image capturing device.

In addition, it may be preferable that the invention further includes a sensor member adapted to sense the external stimulus applied to the test subject or a specific behavior pattern of an operator for taking images and induce an implementation of the image capturing device.

In addition, it may be preferable that the invention further includes a stopper adapted to constantly maintain a position selected for the test subject supporter to be fixed in the darkroom box when the test subject supporter moves in and out of the darkroom box.

In addition, it may be preferable that the invention further includes a lighting device adapted to supply light when the external stimulus is applied to the test subject.

In addition, it may be preferable that the image processing system is adapted to perform at least one function of image processing, display, storage, and analysis for the images taken, wherein the analysis analyzes the images by parameterization, digitization, and two dimensional or three dimensional maps.

### [Advantageous Effects]

The imaging device according to the present invention can take real time images of influence on and changes in an animal while applying an external stimulus in a state in which a part of the animal body protrudes from the darkroom box or changes in the animal due to the external stimulus can be imaged because images before and after the external stimulus is applied are obtainable, and all of which can be statistically analyzed, thus an accuracy of imaging measurement can be improved.

### [Description of Drawings]

FIG. 1 is a block diagram schematically illustrating a structure of an imaging device for animal test subjects according to one embodiment of the present invention.
FIG. 2 is a block diagram schematically illustrating a state in which a test subject supporter protrudes outward from a darkroom box according to one embodiment of the present invention.
FIG. 3 is a perspective view schematically illustrating the imaging device according to one embodiment of the present invention.
FIG. 4 is an enlarged perspective view illustrating a darkroom box according to one embodiment of the present invention.
FIG. 5 is a perspective view schematically illustrating the test subject supporter according to one embodiment of the present invention.
FIG. 6 is a cross-sectional view schematically illustrating a state in which a stopper is installed according to one embodiment of the present invention.
FIG. 7 is a perspective view schematically illustrating an imaging device for animal test subjects according to another embodiment of the present invention.
FIG. 8 is a perspective view schematically illustrating an imaging device for animal test subjects according to still another embodiment of the present invention.

### [Modes of the Invention]

Hereinafter, an imaging device for animal test subjects according to one embodiment of the present invention will be described in detail with reference to the accompanying drawings. An animal serving as a test subject will be taken as an example for convenience of description. Thicknesses of lines and sizes of components may be somewhat exaggerated in the drawing for the sake of clarity and convenience of descriptions.

In addition, terms described below are defined considering functions of the present invention, but the terms may be changed by an intention or a practice of users and operators. Thus, definitions of these terms may have to be made on the basis of the content throughout the specification.

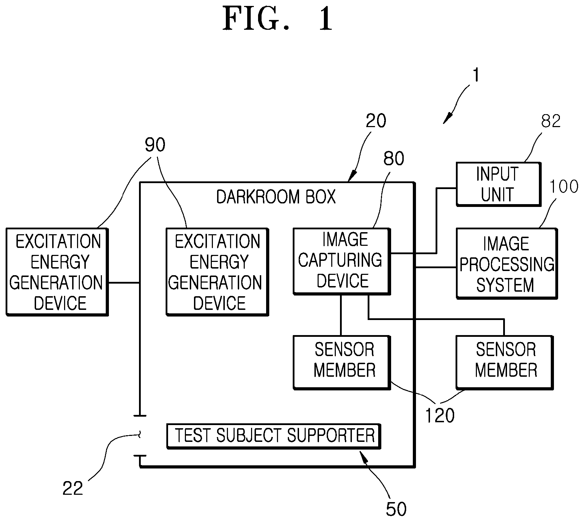

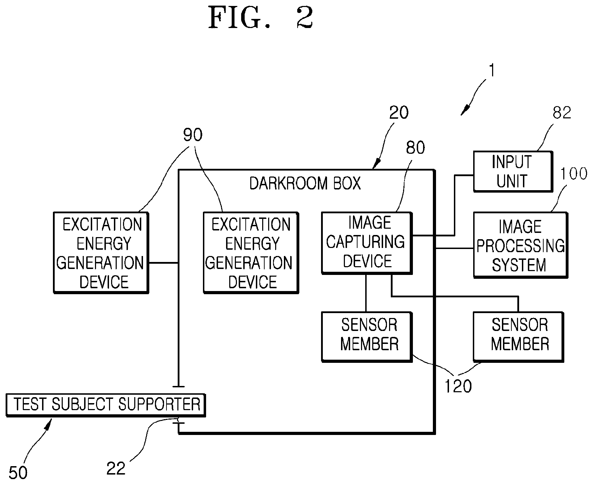

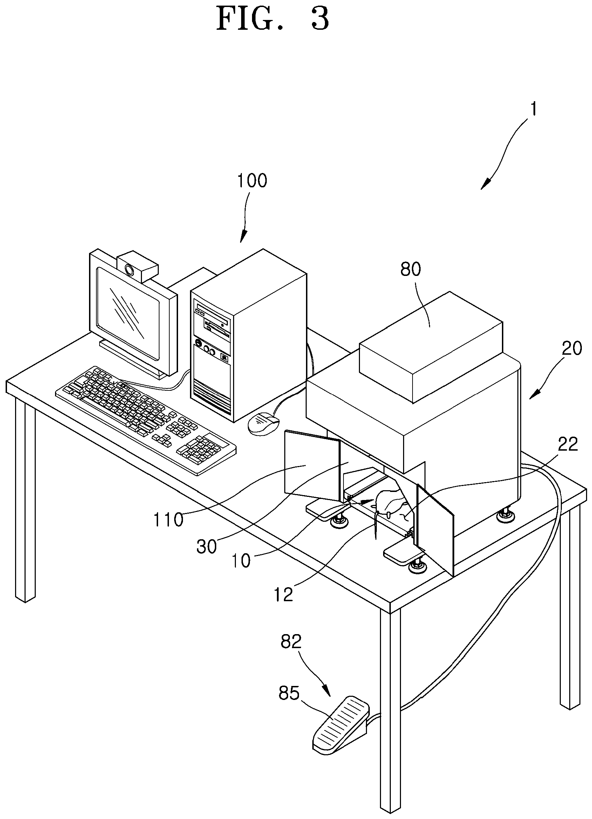

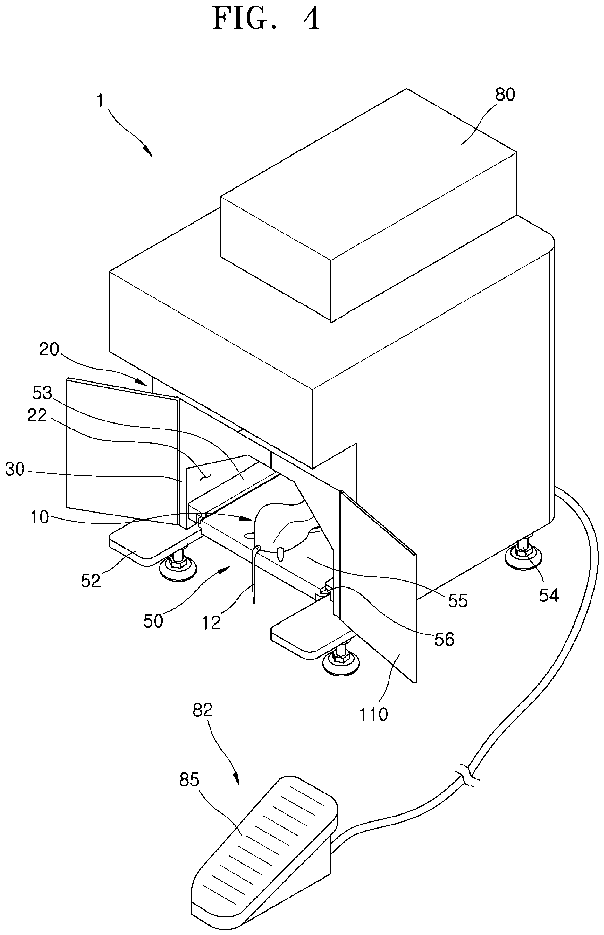

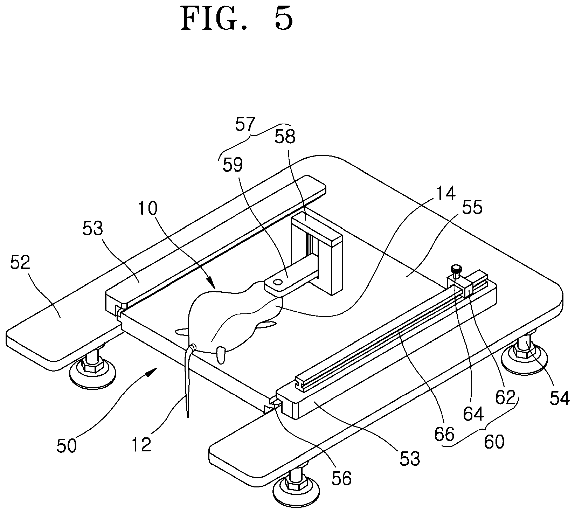

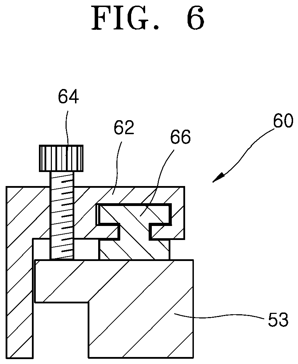

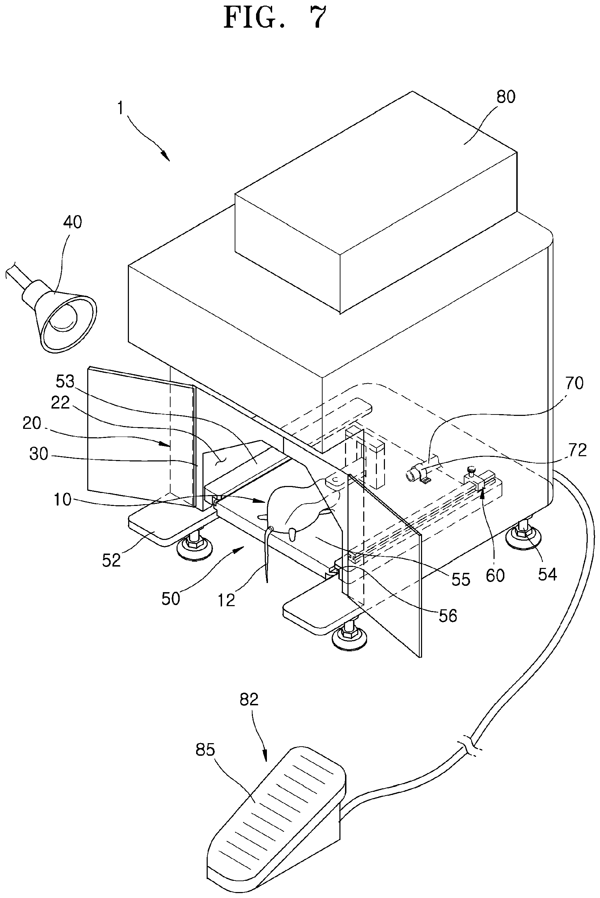

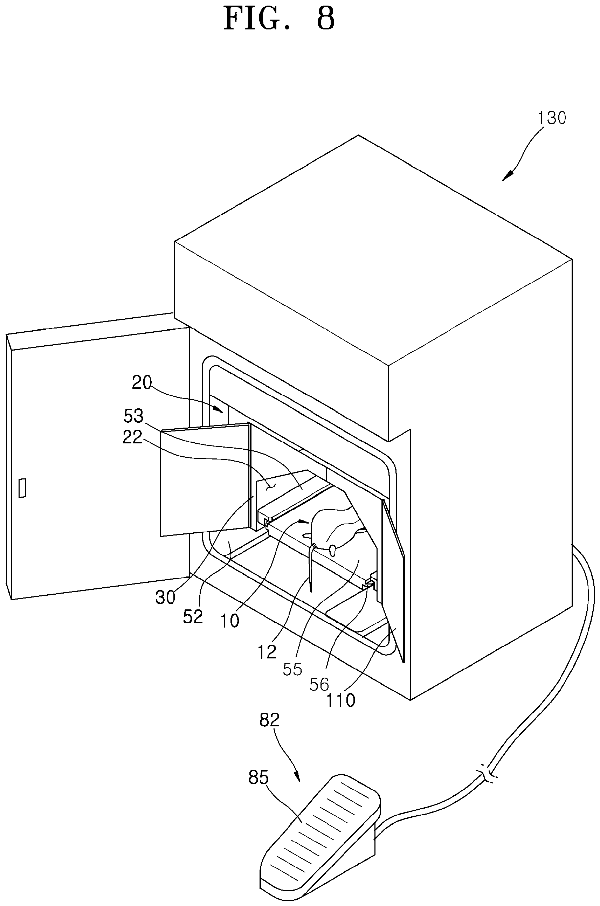

FIG. 1 is a block diagram schematically illustrating a structure of an imaging device for animal test subjects according to one embodiment of the present invention, FIG. 2 is a block diagram schematically illustrating a state in which a test subject supporter protrudes outward from a darkroom box according to one embodiment of the present invention, FIG. 3 is a perspective view schematically illustrating the imaging device for animal test subjects according to one embodiment of the present invention, FIG. 4 is an enlarged perspective view illustrating a darkroom box according to one embodiment of the present invention, FIG. 5 is a perspective view schematically illustrating the test subject supporter according to one embodiment of the present invention, FIG. 6 is a cross-sectional view schematically illustrating a state in which a stopper is installed according to one embodiment of the present invention, FIG. 7 is a perspective view schematically illustrating an imaging device for animal test subjects according to another embodiment of the present invention, and FIG. 8 is a perspective view schematically illustrating an imaging device for animal test subjects according to still another embodiment of the present invention.

As illustrated in FIGS. 1 to 6, an imaging device 1 for animal test subjects according to one embodiment of the present invention includes a darkroom box 20 having an opening 22 through which a part of a test subject 5 to be stimulated protrudes to the outside, a test subject supporter 50 provided to mount the test subject 5 so that a part of the test subject 5 protrudes to the outside, an image capturing device 80 which takes images of the test subject 5 before, after, or while an external stimulus is activated while the external stimulus is applied to a part of the test subject 5 mounted on the test subject supporter 50, and an image processing system 100 for processing the images taken by the image capturing device 80.

The test subject 5 includes a body of an animal 10The imaging device for animal test subjects 1 according to one embodiment of the present invention is a device used when taking images of a living animal 10 in an anesthetized or non-anesthetized state for the purpose of imaging a shape, a structure, a function, etc. of the animal 10.

Besides a mouse, a rat, and a rabbit which are mainly used for research, a mammal such as a dog, a cat, a pig, etc., an amphibian, and the like are included as the animal 10 among test subjects 5 to be put into the imaging device 1 for obtaining images.

When an external stimulus is directly or indirectly applied to a part of a body of the animal 10, the imaging device 1 according to one embodiment of the present invention is used to image responses at the part to which the stimulus is applied and another part to which the stimulus is not applied.

The darkroom box 20 may be formed in various shapes which block external light within the inventive concept and is provided with the opening 22 so that a part of the test subject 5 protrudes to the outside. The darkroom box 20 according to one embodiment of the present invention includes the test subject supporter 50 therein and is formed in a box shape. Through the opening 22, the test subject 5 may move in and out of the darkroom box 20, and the test subject supporter 50 on which the test subject 5 is mounted may move in and out of the darkroom box 20.

After the animal 10 is positioned in the darkroom box 20, a blackout device 30 which manually or automatically adjusts a size of the opening 22 through which external light is incident into the darkroom box 20 is installed.

The blackout device 30 is installed at the darkroom box 20 before or after completing movement of the test subject supporter 50 on which the test subject 5 is mounted into the darkroom box 20. The blackout device 30 is sufficiently open to a size for applying an external stimulus to the animal 10 serving as the test subject 5 mounted on the test subject supporter 50, and the opened size may be adjusted depending on a size of the body of the animal 10 which protrudes outward from the darkroom box 20.

The blackout device 30 is manually operated or automatically operates to adjust a quantity of incident light into the darkroom box 20, and when a tail 12 of the animal 10 used as the test subject 5 protrudes outward from the darkroom box 20, the blackout device 30 may block the incident light into the darkroom box 20 by shielding all other portions besides the tail 12 of the animal 10.

The blackout device 30 is formed in a curtain shape and may perform a manual or automatic operation which laterally opens the opening 22 or closes the opening 22 toward the center. In the case, in which the blackout device 30 automatically operates, the blackout device 30 may be laterally moved by receiving power of a motor in a gear engagement method. Alternatively, various modified implementations are possible for moving the blackout device 30 such as broadening or narrowing the size of the opening 22 by vertically moving the blackout device 30 in a curtain shape, and the like.

Alternatively, the blackout device 30 may be formed in a sheet shape to manually or automatically perform an operation of laterally opening or closing toward the center. In the case in which the blackout device 30 automatically operates, the blackout device 30 may be laterally moved by receiving power of a motor via an engagement method of a rack and pinion gear. Alternatively, various modified implementations are possible for broadening or narrowing the size of the opening 22 by vertically moving the blackout device 30 in a sheet shape, and the like.

The driving force to automatically move the blackout device 30 may employ various types of drive sources such as a cylinder, a linear driving device, a motor, or the like, and power of these drive sources may be transferred to the blackout device 30 via gear engagement, etc.

To block the light from being incident into the darkroom box 20 via the opening 22, at least one of the blackout device 30 and an outer door 110 may be used as needed. That is, the blackout device 30 or the outer door 110 may be installed at the opening 22, or both of the blackout device 30 and the outer door 110 may be installed.

The outer door 110 shields the opening 22 to block external light from being incident into the darkroom box 20. As illustrated in FIGS. 4 and 8, the outer door 110 is rotatably installed at the darkroom box 20 or an extension box 130 which surrounds the darkroom box 20, and is rotated by an operation of an operator to shield a front surface of the opening 22 or minimize an extent to which the opening 22 is open. The outer door 110 is manually opened and closed, but, according to need, the opening and the closing may also be automatically performed by transferring power of a motor to the rotation shaft of the outer door 110. In addition, the outer door 110 may be moved in a rotating method, and may be installed to be slideable in a horizontal direction at an outer side of the darkroom box 20.

As illustrated in FIG. 8, the extension box 130 may be additionally installed in a shape of surrounding an outer side of the darkroom box 20, and the outer door 110 may be installed at each of the darkroom box 20 and the extension box 130. Alternatively, various modified implementations are possible, such as the outer door 110 being installed at only one of the darkroom box 20 or the extension box 130, etc., as needed.

As illustrated in FIG. 7, in another embodiment of the present invention, a lighting device 40 may be installed outside the darkroom box 20. The lighting device 40 may supply light to facilitate work when applying an external stimulus to the body of the animal 10 serving as the test subject 5 which protrudes outward from the darkroom box 20. In addition, it is preferable that an image-taking software of the image capturing device 80 and the image processing system 100 be interlocked with power control of the lighting device 40 to control the power of the lighting device 40 to be turned off during the image-taking of the image capturing device 80 to prevent light from being incident into the darkroom box 20 via the opening 22. Meanwhile, the lighting device 40 may provide a bright work environment for easier work visually when applying an external stimulus to the animal 10.

As illustrated in FIGS. 1 to 6, the test subject supporter 50 may be formed in various shapes in which an animal 10 is mounted so that a part of a body of the animal 10 serving as a test subject 5 protrudes outward from the darkroom box 20 within the inventive concept.

The test subject supporter 50 according to one embodiment allows the animal 10 to be mounted so as not to disturb motion of the animal 10 when taking images of motion of the animal 10. That is, when it is intended to observe motion of the test subject 5 by an external stimulus, tissues expected to move are mounted so as not to be fastened by a fastening device 57 but to be freely moved. To this end, the test subject supporter 50 fastens only a part of a body of the animal 10 which needs to be fastened while permitted movement of other parts of the body of the animal 10 for taking images is not restricted.

The external stimulus includes at least one of a physical stimulus, a chemical stimulus, a temperature change, injecting an external substance into an animal body, and extracting an internal substance of an animal body. That is, the external stimulus may be a stimulus such as a temperature change, a physical or chemical stimulus, injecting a substance into a body, or extracting a substance from a body of the animal 10.

In addition, since the test subject supporter 50 is installed to be movable in and out of the darkroom box 20, various types of embodiments are possible such as moving the test subject supporter 50 to an outside of the darkroom box 20, applying a stimulus to a specific part of the animal 10, moving the test subject supporter 50 back into the darkroom box 20 to take images of the animal 10, and the like.

The animal 10 is mounted on the test subject supporter 50 so that a part of the body of the animal 10 to which the external stimulus is applied protrudes outward from the darkroom box 20. The test subject supporter 50 may or may not protrude outward from the darkroom box 20. In addition, the test subject supporter 50 may be moved in or out of the darkroom box 20 to mount the animal 10 thereon.

The test subject supporter 50 which moves in a sliding method may move in or out of the darkroom box 20, and the animal 10 mounted on the test subject supporter 50 may also be moved in or out of the darkroom box 20 along with the test subject supporter 50.

In addition, the animal 10 may be mounted on the test subject supporter 50 in the anesthetized or non-anesthetized state.

One embodiment describes the imaging device 1 invented to be advantageous for taking images while injecting a contrast medium into a vein of the tail 12 of a mouse or rat employed as the imaging subject animal 10.

The test subject supporter 50 according to one embodiment of the present invention includes a support member 52, guide rails 53, leg members 54, a moving member 55, and a fastening device 57.

The support member 52 having a board shape is installed under the darkroom box 20. An inner space in which the moving member 55 is slideable is provided at an inner side of the support member 52, and guide rails 53 are installed on opposite sides in a width direction of the support member 52 (a lateral direction based on FIG. 5). Two guide rails 53 form a pair and are installed in parallel, and guide protrusions 56 which protrude on opposite sides of the moving member 55 are inserted between the guide rails 53 and the support member 52 to be slideable.

In addition, installation places and the number of the support member 52 and the guide rails 53 may be modified depending on the size of the moving member 55. For instance, in the case in which the size of the moving member 55 is enlarged, the support member 52 and the guide rails 53 may be formed to be two or more and installed beneath the moving member 55.

The leg members 54 installed under the support member 52 are adjustable in length. The leg members 54 may be installed under the darkroom box 20 or the support member 52. Since the protrusion lengths of the leg members 54 are adjustable, a height of the darkroom box 20 and the test subject supporter 50 are adjustable.

When applying an external stimulus to the tail 12 of the animal 10 which protrudes outward from the darkroom box 20, the lengths of the leg members 54 may be adjusted to provide a sufficient space between the tail 12 and a floor or between the tail 12 and a desk on which the darkroom box 20 is put for convenient hand motion of an operator.

A space suitable for applying an external stimulus is securable depending on a size of the animal 10 which protrudes outward from the darkroom box 20, since the space between the desk and the darkroom box 20 is adjustable by adjusting the lengths of the leg members 54. The leg members 54 may be installed at an inner side or an outer side, or at the support member 52 of the test subject supporter 50, but it is advantageous to be installed at the outer side of the darkroom box 20 or under the support member 52 for freely adjusting the height.

The moving member 55 is formed in a board shape on which the animal 10 is put, and guide protrusions 56 are provided on opposite sides of the moving member 55 to guide the moving member 55 in a horizontal direction through the guide rails 53. The moving member 55 of the test subject supporter 50 is installed in a sliding method to be freely moved in and out of the darkroom box 20, and is slid and pulled out of the darkroom box 20 when mounting the animal 10 such as a mouse on the moving member 55.

The fastening device 57 may be formed in various shapes within the inventive concept, which fasten or support a part of a body of the animal 10 to restrict unexpected motion of the animal 10. In addition, the fastening device 57 may be moved and attached at a different position, and is detachable.

The fastening device 57 fastens only a part of a body of an animal to restrict motion of a region of the body to be imaged by the image capturing device 80. In addition, an installation position of the fastening device 57 may be adjustable to match various sizes of animals 10 since the fastening device 57 may be moved and attached in the test subject supporter 50 and is detachable.

The fastening device 57 according to one embodiment includes a fastening body 58 fixed to the moving member 55 of the test subject supporter 50, and a pressing member 59 which vertically moves along the fastening body 58 and grabs the animal 10. The fastening body 58 is fitted and coupled to the moving member 55 or bolt-coupled thereto, and an installation position may be changed as needed. The fastening body 58 is perpendicularly installed on the moving member 55.

The pressing member 59 may be manually operated or automatically operates along the fastening body 58 and presses the animal 10 at a predetermined force to restrict a movement of the animal 10. The pressing member 59 moves vertically along the fastening body 58, and, when the pressing member 59 is moved manually, an engaging bolt (not shown) which vertically passes through the fastening body 58 presses an upper side of the pressing member 59. Therefore, the pressing member 59, which presses an animal, is constrained from moving upward. An operator may constrain the pressing member 59 from moving upward by moving the pressing member 59 by hand to press the animal 10 and rotating the engaging bolt to press an upper side of the pressing member 59.

In the case in which the pressing member 59 moves automatically, the pressing member 59 is installed to be vertically slideable along the fastening body 58. Protrusions protrude on opposite sides of the pressing member 59 in a horizontal direction, the protrusions, which protrude from the pressing member 59, are inserted into the fastening body 58, and thus the pressing member 59 may vertically slide along the fastening body 58. A pressing device such as a cylinder is installed between an upper side of the pressing member 59 and the fastening body 58 to move the pressing member 59 downward. A rack gear is additionally installed at the pressing member 59, a pinion gear is installed at a rotating axis of a motor, and the rack gear and the pinion gear are engaged to each other to move the pressing member 59 to an extent of a predetermined length. Besides the above, the pressing member 59 may automatically move using various types of driving apparatuses.

Therefore, when the animal 10 is mounted on the test subject supporter 50, the animal 10 may be assisted in being mounted to be immobile by a device which fastens a part of a body of the animal 10 such as the fastening device 57.

When mounting the animal 10 such as a mouse on the test subject supporter 50, the animal 10 is mounted so that more than one third of the tail 12 of the animal 10 protrudes from the moving member 55 of the test subject supporter 50. Here, the animal 10 is fastened on the test subject supporter 50 to facilitate the mounting of the animal 10. For example, a part of the body of the animal 10 such as a head 14, teeth, a leg, a torso, or the like may be fastened by the moving member 55 of the test subject supporter 50. The fastening device 57 may be installed at the moving member 55 of the test subject supporter 50 so as to be easily moved to a position or be removed. When mounting the animal 10 by the fastening device 57 is completed, the moving member 55 of the test subject supporter 50 is moved into the darkroom box 20.

The imaging device 1 includes a stopper 60 which constantly maintains a position selected for the test subject supporter 50 to be fixed in the darkroom box 20 when the test subject supporter 50 moves in and out of the darkroom box 20.

Various types of stopper devices may be used within the inventive concept to select a position of the moving member 55 of the test subject supporter 50 so that the image capturing device 80 may take optimum images of a whole or a part of a body of the animal 10 and to restrict a movement of the moving member 55 of the test subject supporter 50 at a selected position.

The stopper 60 performs a function of stopping the moving member 55 at a desired or selected position while it is advancing into the darkroom box 20. The stopper 60 is manually operated or automatically operates to restrict a movement of the moving member 55 so as to quickly take images of the animal 10 upon applying an external stimulus.

The stopper 60 according to one embodiment includes a stopper body 62, an adjusting knob 64, and a base member 66. The base member 66 is fixedly installed to the guide rail 53, and the stopper body 62 is slid along the base member 66 to stop at the position where the moving member 55 is supposed to be stopped. Thus, a movement of the moving member 55 is restricted as the moving member 55 is caught by the stopper body 62 while moving along the guide rail 53.

The adjusting knob 64 is rotatably installed at the stopper body 62 and restricts a movement of the stopper body 62 since the adjusting knob 64 is rotated to come into contact with the guide rail 53. Thus, a movement of the moving member 55, which moves along the guide rail 53, is restricted.

The base member 66 is fixed on the guide rail 53, and stopper grooves are formed on opposite sides of the base member 66. The stopper body 62 is provided with protrusions inserted into the stopper grooves provided on opposite sides of the base member 66, and moves along the base member 66 in a rod shape. As the protrusions, which extend downward from the stopper body 62, are positioned at the side surface of the guide rail 53, the protrusions may restrict the moving member 55 which moves along the guide rail 53

An inhalation anesthesia apparatus for animals may be installed at the darkroom box 20 or the test subject supporter 50. The inhalation anesthesia apparatus for animals according to one embodiment includes a tube 70 and a tube support member 72.

The tube 70 through which an anesthetic gas is supplied for inhalation anesthesia for the animal 10 is installed at the test subject supporter 50, and a movement of the tube 70 is restricted by the tube support member 72. The tube 70 according to one embodiment is positioned at the moving member 55 of the test subject supporter 50, and the tube support member 72, which surrounds an outer side of the tube 70, is fixed at the moving member 55.

Various types of image capturing devices 80 may be used within the inventive concept to take images of the animal 10 mounted on the test subject supporter 50 while applying an external stimulus to a part of the animal immediately before, immediately after, or at the same time as the application of the external stimulus. In addition, the image capturing device 80 may facilitate acquisition of images even when external light is incident via the opening 22.

The image capturing device 80 according to one embodiment is formed as a combination of at least one of an optical image capturing device, X-rays, computerized tomography (CT), and magnetic resonance imaging (MRI).

The above-described image capturing device 80 may acquire time-series images in real time or images which are not time-series at the same time as an external stimulus. The time-series images refer to images taken in a time sequence, and is also referred to as a video. The images, which are not time-series images, refer to various forms of photographic images including snapshot images.

In addition, the image capturing device 80 is capable of taking images before, after, or at the same time as the application of the external stimulus to the animal 10. That is, the image capturing device 80 may take images of a time-series array or images which are not a time-series array at a desired time from before to after the application of the external stimulus.

In addition, the image capturing device 80 may increase measurement accuracy by providing an image for assuring that the test subject supporter 50 is put at a suitable position for imaging before main imaging.

Various types of input devices may be used as an input unit 82 which receives operational signals for operating the image capturing device 80. The input unit 82 according to one embodiment uses a foot switch 85 which is operable by foot. The foot switch 85 operated by a foot of an operator includes a function of operating the image capturing device 80. The image capturing device 80 may be operated by hands of the operator, and may be operated using the foot switch 85 operated by the foot of the operator when the operator works with his or her hands to apply an external stimulus to the animal 10.

Various types of devices may be used as excitation energy generation devices 90 which emit energy for the image capturing device 80 to acquire images within the inventive concept.

The excitation energy generation devices 90 are preferably installed at least at one place of an inside or outside of the darkroom box 20. In addition, the excitation energy generation devices 90 may generate a sufficient energy so that the image capturing device 80 is not hindered in acquiring images even when external light is incident to a part of the darkroom box 20.

The excitation energy generation devices 90 are devices which generate various types of light or energy such as light energy, visible rays, X-rays, etc. within the inventive concept and emit energy for the image capturing device 80 to acquire images.

As a use example of the imaging device 1 in the case of using a contrast medium as the external stimulus, a contrast medium for near-infrared rays is injected to a blood vessel of a tail of the animal 10 which protrudes outward from the darkroom box 20, to which a light emitting diode (LED) of near-infrared rays among the excitation energy generation devices 90 installed in the darkroom box 20 is operated and responds to the contrast medium for near-infrared ray injected to the body of the animal 10.

In the image capturing device 80 which take images of fluorescent images of the contrast medium, the image capturing device 80 of the near-infrared rays may take images of the time-series images. As an advantage of the imaging device 1, image taking may be started immediately before, immediately after, or simultaneously with an injection of the contrast medium, and thus a pattern with which the contrast medium spreads in the body is accurately grasped by taking time-series images.

Various types of image processing systems may be used as the image processing system 100 which processes images taken by the image capturing device 80 within the inventive concept. The image processing system 100 performs at least one function of image processing, display, storage, and analysis for the images taken by the image capturing device 80. The analysis of images includes analyzing images by parameterization, digitization, and two dimensional or three dimensional maps. In addition, functions of the image processing system 100 may be achieved in a single device or multiple devices.

In addition, it is preferable that image processing, display, storage and analysis of the image processing system 100 is performed by a single device or multiple devices, and the images taken by the image capturing device 80 are stored after being displayed at the image processing system 100. The image processing system 100 may analyze the images and perform functions such as parameterization, digitization, quantification, or mapping.

The imaging device 1 includes a sensor member 120 which senses an external stimulus applied to the animal 10 or a specific behavior pattern of an operator for taking images, and induces implementation of the image capturing device 80. The sensor member 120 is installed at least at one place of an inner side and outer side of the darkroom box, and a plurality of sensors different from each other may be installed as needed.

Hereinafter, an operational state of the imaging device 1 according to one embodiment will be described in detail with reference to the accompanying drawings.

The moving member 55 of the test subject supporter 50 is pulled out of the darkroom box 20. A mouse serving as a subject animal 10 is pre-anesthetized before being mounted on the test subject supporter 50, and is put on the test subject supporter 50. Alternatively, the animal 10 may be anesthetized by inhalation anesthesia in the darkroom box 20 when the tube 70 for inhalation anesthesia is connected to the darkroom box 20.

A head 14 of the animal 10 is fastened by the fastening device 57 so that the animal 10 is mounted to be immobile and comfortable. Here, the tube 70 for inhalation anesthesia may be positioned together with the fastening device 57.

Surgery on the mouse serving as the subject animal 10 may have been conducted in advance for imaging, and the mouse is mounted so that one third or more of a tail 12 protrudes outward from the test subject supporter 50 for injecting into a vein of the tail 12.

In addition, the moving member 55 of the test subject supporter 50 is moved into the darkroom box 20. At this point, a position to which the animal 10 is moved is selected to be a position in which a most interesting part to be imaged in the body of the animal 10 is perpendicularly in line with the image capturing device 80. A position of the stopper body 62 is adjusted so that the moving member 55 is stopped at the selected position. The reason, why the position at which the animal 10 is put is set by the stopper 60, is to start taking images upon pushing the moving member 55 of the test subject supporter 50 into the darkroom box 20 immediately after injecting at the outside of the darkroom box 20, when injecting into the vein of the tail 12 of the animal 10. When images are taken using the stopper 60, taking images may be achieved in a quick and accurate manner since the animal is quickly moved to the position which is preset for the taking of images.

Depending on preference of an operator, an external stimulus may be applied after positioning the test subject supporter 50 on which the animal 10 is mounted to the position in the darkroom box 20 where the image taking is to be conducted. Alternatively, a part of the test subject supporter 50, on which the animal 10 is mounted, is pulled out of the darkroom box 20 for applying the external stimulus, and then the moving member 55 of the test subject supporter 50 is pushed into the darkroom box 20 to start taking images immediately after the external stimulus, or after a predetermined amount of time passes.

When taking images, the image capturing device 80 may be adjusted by the foot switch 85 for allowing hands of the operator to easily apply the external stimulus. Images may be taken in real time and time-series, or by each shot of images. Once the images are taken, the image processing system 100 may display or store the images during or after the image taking. An image analysis device in the image processing system 100 may be the same device which displays and stores the images, or may be installed in another apparatus. Accordingly, the images may be checked or analyzed after taking the images through the image processing system 100.

The imaging device 1 according to one embodiment of the present invention may facilitate taking images when imaging a contrast medium or pharmacokinetics, particularly when taking time-series images, and when taking images immediately after injecting the contrast medium.

A conventional imaging device uses a darkroom box 20 sealed after putting an animal 10 therein and requires some time before taking images after injecting a contrast medium including such time as mounting the animal 10, closing an opening 22 of the darkroom box 20, implementing a start operation on a computer, and the like, and thus, in this case, images immediately after injecting a contrast medium may not obtained. Particularly, when pharmacokinetics of a contrast medium is very dynamic or quickly flows, many images immediately after injecting a drug may be missed. The imaging device 1 according to one embodiment of the present invention is capable of taking real time images without loss for an external stimulus which dynamically acts, and is a useful invention also in the case in which images on real time changes in a living body due to an external stimulus such as physical stimuli including touching, cooling, heating, etc. and a chemical stimulus are required.

As described above, according to the embodiment of the present invention, since real-time images of the influence on and changes in the animal 10 while applying an external stimulus in a state in which a part of a body of the animal 10 protrudes outward from the darkroom box 20 are obtainable as well as the images before and after the external stimulus is applied, changes caused by the external stimulus may be imaged, statistically analyzed, and thereby accuracy of an imaging measurement can be improved.

While the present invention has been described in connection with limited embodiments illustrated in the drawings, the above-described embodiments should be considered in a descriptive sense only and not for purposes of limitation, and it should be obvious to those skilled in the art that various modifications and other embodiments may be made without departing from the scope of the appended claims.

## Claims

1. An imaging device (1) for animal test subjects, comprising:
a darkroom box (20) provided with an opening (22) adapted to allow a part of a test subject (5) to protrude outward from the darkroom box (20) while a remaining part of the test subject is positioned inside the darkroom box (20);
a test subject supporter (50) adapted for mounting a test subject (5) thereon;
an image capturing device (80) adapted to take images of the test subject (5) mounted on the test subject supporter (50) inside the darkroom box (20) while an external stimulus is applied to the part of the test subject (5) positioned outside the darkroom box (20),
**characterized in that**
the imaging device (1) further comprises an image processing system (100) adapted to process the images taken by the image capturing device (80),
wherein the darkroom box (20) has a blackout device (30) installed at the opening (22), the blackout device (30) being adapted to block incident light into the darkroom box (20) by shielding other portions of the test subject (5) besides the protruding part of the test subject (5), and
wherein the blackout device is adapted to manually or automatically adjust, depending on a size of the body of the test subject (10) which protrudes outward from the darkroom box (20), a size of the opening (22).

2. The imaging device (1) of claim 1, wherein the test subject supporter (50) is installed to be movable in and out of the darkroom box (20).

3. The imaging device (1) of claim 2, wherein the test subject supporter (50) moves in a sliding manner.

4. The imaging device (1) of claim 1, wherein the test subject supporter (50) includes a fastening device (57) adapted to fasten the part of the test subject in order to restrict unexpected motion of the animal body, and the fastening device (57) is adapted to be moved and attached at a different position, and is detachable.

5. The imaging device (1) for claim 1, wherein the image capturing device (80) includes at least one of an optical image capturing device, X-rays, computerized tomography (CT), and magnetic resonance imaging (MRI).

6. The imaging device (1) of claim 1, further comprising an excitation energy generation device (90) adapted to emit energy for the image capturing device (80) to acquire an image and which is installed at least at one place of an inside or outside of the darkroom box (20).

7. The imaging device (1) of claim 1, further comprising an outer door (110) adapted to shield the opening (22) to block external light from being incident into the darkroom box (20).

8. The imaging device (1) of claim 1, further comprising an input unit (82) adapted to receive operational signals for operating the image capturing device (80).

9. The imaging device (1) of claim 1, further comprising a sensor member (120) adapted to sense the external stimulus applied to the test subject (5) or a specific behavior pattern of an operator for taking images and to induce an implementation of the image capturing device (80).

10. The imaging device (1) of claim 1, further comprising a stopper (60) adapted to constantly maintain a position selected for the test subject supporter (50) to be fixed in the darkroom box (20) when the test subject supporter (50) moves in and out of the darkroom box (20).

11. The imaging device (1) of claim 1, further comprising a lighting device (40) adapted to supply light when the external stimulus is applied to the test subject (5).

12. The imaging device (1) of claim 1, wherein the image processing system (100) is adapted to perform at least one function of image processing, display, storage, and analysis for the images taken, wherein the analysis analyzes the images by parameterization, digitization, and two dimensional or three dimensional maps.

## Patentansprüche

1. Bildgebungsvorrichtung (1) für Tiertestobjekte, die Folgendes umfasst:
einen Dunkelkammerkasten (20), der mit einer Öffnung (22) versehen ist, welche so angepasst ist, dass ein Teil eines Testobjekts (5) aus dem Dunkelkammerkasten (20) herausragen kann, während ein verbleibender Teil des Testobjekts im Inneren des Dunkelkammerkastens (20) angeordnet ist;
einen Testobjektträger (50), auf dem ein Testobjekt (5) befestigt werden kann;
eine Bildaufnahmevorrichtung (80), die angepasst ist, um innerhalb des Dunkelkammerkastens (20) Bilder des auf dem Testobjektträger (50) angebrachten Testobjekts (5) aufzunehmen, während auf den außerhalb des Dunkelkammerkastens (20) positionierten Abschnitts des Testobjekts (5) ein externer Stimulus ausgeübt wird,
**dadurch gekennzeichnet, dass**
die Bildgebungsvorrichtung (1) ferner ein Bildverarbeitungssystem (100) umfasst, welches zur Verarbeitung der von der Bildaufnahmevorrichtung (80) aufgenommenen Bilder angepasst ist,
wobei der Dunkelkammerkasten (20) eine an der Öffnung (22) installierte Verdunkelungsvorrichtung (30) aufweist, wobei die Verdunkelungsvorrichtung (30) angepasst ist, in den Dunkelkammerkasten (20) einfallendes Licht zu blockieren, indem sie andere Abschnitte des Testobjekts (5) neben dem herausragenden Abschnitt des Testobjekts (5) abschirmt, und
wobei die Verdunkelungsvorrichtung angepasst ist, um die Größe der Öffnung (22) in Abhängigkeit von der Größe des aus dem Dunkelkammerkasten (20) herausragenden Körpers des Testobjekts (10) manuell oder automatisch einzustellen.

2. Bildgebungsvorrichtung (1) nach Anspruch 1, wobei der Testobjektträger (50) installiert ist, um in den Dunkelkammerkasten (20) hinein und aus diesem heraus bewegt zu werden.

3. Bildgebungsvorrichtung (1) nach Anspruch 2, wobei sich der Testobjektträger (50) auf eine gleitende Art und Weise bewegt.

4. Bildgebungsvorrichtung (1) nach Anspruch 1, wobei der Testobjektträger (50) eine Befestigungsvorrichtung (57) umfasst, welche angepasst ist, den Abschnitt des Testobjekts zu befestigen, um unerwartete Bewegungen des Tierkörpers einzuschränken, und wobei die Befestigungsvorrichtung (57) angepasst ist, um bewegt und an einer anderen Position angebracht zu werden, und wobei diese abnehmbar gestaltet ist.

5. Bildgebungsvorrichtung (1) nach Anspruch 1, wobei die Bilderfassungsvorrichtung (80) mindestens eines von einer optischen Bilderfassungsvorrichtung, Röntgenstrahlen, Computertomographie (CT) und Magnetresonanztomographie (MRI) umfasst.

6. Bildgebungsvorrichtung (1) nach Anspruch 1, ferner mit einer Anregungsenergieerzeugungsvorrichtung (90), welche angepasst ist, um Energie für die Bildaufnahmevorrichtung (80) zu emittieren, um ein Bild zu erfassen, und die an mindestens einer Stelle innerhalb oder außerhalb des Dunkelkammerkastens (20) installiert ist.

7. Bildgebungsvorrichtung (1) nach Anspruch 1, ferner mit einer Außentür (110), welche so angepasst ist, dass sie die Öffnung (22) abschirmt, um zu verhindern, dass Licht von außen in den Dunkelkammerkasten (20) einfällt.

8. Bildgebungsvorrichtung (1) nach Anspruch 1, ferner mit einer Eingabeeinheit (82), welche so angepasst ist, dass sie Betriebssignale zum Betreiben der Bildaufnahmevorrichtung (80) empfängt.

9. Bildgebungsvorrichtung (1) nach Anspruch 1, ferner mit einem Sensorelement (120), welches so beschaffen ist, dass es den auf das Testobjekt (5) ausgeübten externen Stimulus oder ein bestimmtes Verhaltensmuster eines Bedieners zur Aufnahme von Bildern erfasst und eine Ausführung der Bildaufnahmevorrichtung (80) veranlasst.

10. Bildgebungsvorrichtung (1) nach Anspruch 1, ferner mit einem Stopper (60), welcher angepasst ist, um eine für die Fixierung des Testobjektträgers (50) in dem Dunkelkammerkasten (20) ausgewählte Position konstant dann beizubehalten, wenn sich der Testobjektträger (50) in den Dunkelkammerkasten (20) hinein und aus diesem heraus bewegt.

11. Bildgebungsvorrichtung (1) nach Anspruch 1, ferner mit einer Beleuchtungsvorrichtung (40), welche angepasst ist, um Licht dann zu liefern, wenn der externe Stimulus auf das Testobjekt (5) angewendet wird.

12. Bildgebungsvorrichtung (1) nach Anspruch 1, wobei das Bildverarbeitungssystem (100) angepasst ist, um mindestens eine Bildverarbeitungsfunktion, eine Anzeigefunktion, eine Speicherungsfunktion und eine Analysefunktion für die aufgenommenen Bilder auszuführen, wobei die Analyse die Bilder durch Parametrisierung, Digitalisierung und anhand von zweidimensionalen oder dreidimensionalen Karten analysiert.

## Revendications

1. Dispositif d'imagerie (1) pour sujets animaux de test, comprenant :
une boîte de chambre noire (20) munie d'une ouverture (22) configuré pour permettre à une partie d'un sujet de test (5) de faire saillie vers l'extérieur de la boîte de chambre noire (20) tandis qu'une partie restante du sujet de test est positionnée à l'intérieur de la boîte de chambre noire (20) ;
un élément de support de sujet de test (50) configuré pour monter sur celui-ci un sujet de test (5) ;
un dispositif de capture d'image (80) configuré pour prendre des images du sujet de test (5) monté sur l'élément de support de sujet de test (50) à l'intérieur de la boîte de chambre noire (20) tandis qu'un stimulus externe est appliqué à la partie du sujet de test (5) positionnée à l'extérieur de la boîte de chambre noire (20),
**caractérisé en ce que** :
le dispositif d'imagerie (1) comprend de plus un système de traitement d'image (100) adapté de façon à traiter les images prises par le dispositif de capture d'image (80),
dans lequel la boîte de chambre noire (20) comporte un dispositif d'occultation (30) installé au niveau de l'ouverture (22), le dispositif d'occultation (30) étant configuré pour empêcher la lumière incidente d'entrer dans la boîte de chambre noire (20) par constitution d'un écran vis-à-vis d'autres parties du sujet de test (5) en dehors de la partie saillante du sujet de test (5), et
dans lequel le dispositif d'occultation est configuré pour être ajusté manuellement ou automatiquement, en fonction de la taille du corps du sujet de test (10) qui fait saillie vers l'extérieur de la boîte de chambre noire (20), et d'une taille de l'ouverture (22).

2. Dispositif d'imagerie (1) selon la revendication 1, dans lequel l'élément de support de sujet de test (50) est installé pour pouvoir rentrer dans la boîte de chambre noire (20) et à sortir de celle-ci.

3. Dispositif d'imagerie (1) selon la revendication 2, dans lequel l'élément de support de sujet de test (50) se déplace de manière coulissante.

4. Dispositif d'imagerie (1) selon la revendication 1, dans lequel l'élément de support de sujet de test (50) comprend un dispositif de fixation (57) configuré pour fixer la partie du sujet de test afin de restreindre un mouvement imprévu du corps de l'animal, et le dispositif de fixation (57) est configuré pour être déplacé et fixé dans une position différente, et est détachable.

5. Dispositif d'imagerie (1) selon la revendication 1, dans lequel le dispositif de capture d'image (80) comprend au moins l'un parmi un dispositif de capture d'image optique, des rayons X, la tomographie informatisée (CT), et l'imagerie à résonance magnétique (IRM).

6. Dispositif d'imagerie (1) selon la revendication 1, comprenant de plus un dispositif de génération d'énergie d'excitation (90) configuré pour émettre de l'énergie pour le dispositif de capture d'image (80) afin d'acquérir une image, et qui est installé au moins en un endroit de l'intérieur ou de l'extérieur de la boîte de chambre noire (20) .

7. Dispositif d'imagerie (1) selon la revendication 1, comprenant de plus une porte extérieure (110) configuré pour occulter l'ouverture (22) afin d'empêcher la lumière externe d'être incidente dans la boîte de chambre noire (20) .

8. Dispositif d'imagerie (1) selon la revendication 1, comprenant de plus une unité d'entrée (82) configuré pour recevoir des signaux d'actionnement pour actionner le dispositif de capture d'image (80).

9. Dispositif d'imagerie (1) selon la revendication 1, comprenant de plus un élément de capteur (120) configuré pour capter le stimulus externe appliqué au sujet de test (5) ou un mode de comportement spécifique d'un opérateur pour prendre des images et pour induire une mise en œuvre du dispositif de capture d'image (80).

10. Dispositif d'imagerie (1) selon la revendication 1, comprenant de plus un élément d'arrêt (60) configuré pour maintenir de façon constante une position sélectionnée pour l'élément de support de sujet de test (50) devant être fixé dans la boîte de chambre noire (20) lorsque l'élément de support de sujet de test (50) rentre dans la boîte de chambre noire (20) et sort de celle-ci.

11. Dispositif d'imagerie (1) selon la revendication 1, comprenant de plus un dispositif d'éclairage (40) configuré pour délivrer de la lumière lorsque le stimulus externe est appliqué au sujet de test (5).

12. Dispositif d'imagerie (1) selon la revendication 1, dans lequel le système de traitement d'image (100) est configuré pour exécuter au moins une fonction parmi le traitement d'image, l'affichage, le stockage et l'analyse pour les images prises, l'analyse analysant les images par paramétrisation, numérisation, et cartographie en deux dimensions ou en trois dimensions.
